# EUROPEAN PATENT SPECIFICATION

(11) **EP 1 243 236 B1**
(45) Date of publication and mention of the grant of the patent: **07.07.2004**
(21) Application number: 02380064.2
(22) Date of filing: 20.03.2002
(51) Int. Cl.: A61F 9/013

(54) **Suction ring for microkeratomes**
Saugring für Mikrokeratome
Anneau de succion pour microkératomes

(30) Priority: 21.03.2001 ES 200100652
(43) Date of publication of application: 25.09.2002
(73) Proprietor: Novosalud S.L., 46007 Valencia (ES)
(72) Inventor: Gutierrez Ortega, Ramon A., 46007 Valencia (ES); Castello Pico, Vicente, 46007 Valencia (ES); Sanz Amoros, Emilio, 46007 Valencia (ES); Sabate Calatayud, Jorge, 46007 Valencia (ES); Santos Pacheco, Salvador, 46007 Valencia (ES); Santatecla Carro, Félix, 46007 Valencia (ES); Vilaseca Buitrago, Emilio, 46007 Valencia (ES); Mares Anton, Javier, 46007 Valencia (ES); Rubira Fernandez, José, 46007 Valencia (ES)
(74) Representative: Maldonado Jordan, Julia

(56) References cited:
- WO-A-92/10152
- WO-A-98/27901
- WO-A-99/03433
- WO-A-99/26568
- FR-A- 2 789 299
- US-A- 4 173 980
- US-A- 5 658 303

## Description

### OBJECT OF THE INVENTION

The present invention refers to a suction ring which uses the depression generated by a vacuum pump connected through a flexible pipe to fix itself to the eyeball and thereby function as the support and guide for the microkeratome cutting blade.

The object of the invention is to improve the ring's capacity to stick to the eyeball and improve the connection of the cutting blade to the suction ring itself.

### BACKGROUND TO THE INVENTION

The entity applying in this case is actually the holder of the Invention Patent with Application No. 9801787 (EP-A-1 147 754) which describes a microkeratome which, among other elements that are not presently of interest, includes a suction ring with a cylindrical outer wall, with a partially incarcerated and bevelled upper opening, with a frontal and lateral cannula for connection, after being fitted to the eyeball, to the vacuum source that will ensure that the said ring may remain perfectly stable on the eyeball while the cutting blade works upon the cornea.

Furthermore, the aforementioned ring also has a dovetail guide at the front, for the longitudinal displacement of the cutting blade whose base is configured according to the said guide.

Moreover, this is the usual configuration of all known suction rings for microkeratomes to date.

Obviously, the extent to which the suction ring is attached to the eyeball depends on the extent to which both elements, ring and eyeball, are adapted to each other. Similarly, the stability of the cutting blade on the said ring also depends on the connection length between the tracks of the suction ring and the cutting blade.

Document WO-A-9 210 152 discloses a suction ring according to the preamble of claim 1.

### DESCRIPTION OF THE INVENTION

The suction ring proposed in the present invention has been conceived and structured with a view to enhancing the two aforementioned aspects, i.e. to improving the ring's capacity to attach itself to the eyeball and to improving the connection between the cutting blade and the dovetail on the suction ring.

To this end, in practice and according to the characteristics of the invention, it has been decided that the side wall of the ring, instead of the classical cylindrical configuration, should have a truncated conical shape, diverging towards the side that is to be attached to the eyeball. This means there is a greater surface of the aforementioned ring in contact with the eyeball and therefore a higher level of fixation between these elements.

In accordance with another of the characteristics of the invention, it has been decided that the ring shall include, on its front edge, a perimeter expansion that will increase the surface of the front side. At the same time, the perimeter of the front and the protruding side shall bulge over the tips of the grooves or scores of the dovetail guide. This shall in turn mean that the magnitude of the sectors of the guides, i.e. the sectors of the ring and the blade that are interconnected at any given time, shall also be widened. Therefore the stability of the cutting blade on the suction ring will be substantially enhanced.

### DESCRIPTION OF THE DRAWINGS

In order to complement the description that is being made and with the aim of allowing a better understanding of the characteristics of the invention, according to an example of the preferred embodiment of same, we include a set of drawings of an illustrative and non-restrictive nature, representing the following:
Figure No. 1.- Shows a perspective view of a traditional suction ring for microkeratomes.
Figure No. 2.- Shows, in a similar view to Figure 1, a ring made in accordance with the object of the present invention, exaggerating its shape so as to allow the reader to contrast clearly and visually the effects achieved by the aforementioned shape.
Figure No. 3.- Shows a ground view of a traditional ring.
Figure No. 4.- Shows a similar view as in the previous figure, but of the ring that is the object of the present invention, also exaggerating its shape in order to show clearly the effects achieved by same.
Figure No. 5. - Shows a side elevation of a specific practical embodiment of a suction ring for microkeratomes in accordance with the invention.
Figure No. 6. - Shows a ground view of the previous figure.
Figure No. 7.- Shows, finally, a side elevation and diameter section of the ring shown in Figure 5, from a viewpoint that is displaced 90° with respect to the aforementioned figure.

### PREFERRED EMBODIMENT OF THE INVENTION

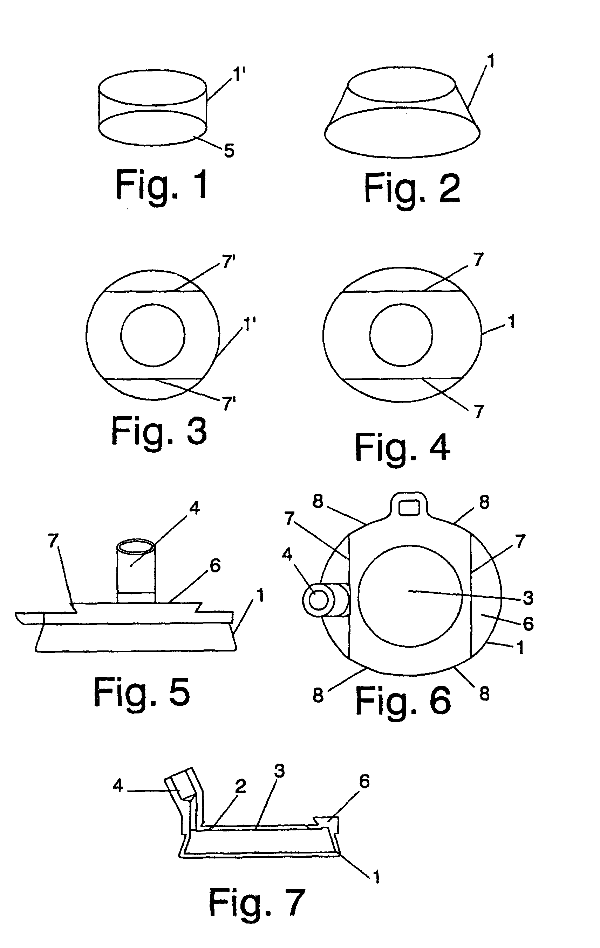

In view of the figures described and particularly of figures 5 to 7, we can see that the suction ring proposed by the present invention is composed, as is the case with any conventional ring, of a pipe (1) whose front opening (2) is noticeably incarcerated, with an inner bevel (3) allowing it to be attached to the front of the eyeball, whereas this pipe (1) has a cannula (4) to connect it, by means of a flexible tube, to a vacuum pump.

As regards the invention itself, the side wall of the body (1) has a truncated conical configuration, noticeably diverging towards the posterior edge of the ring, which is to be attached to the eyeball, as we can see in Figure No. 7. Therefore, as it is obvious if we compare Figures 1 and 2, while a conventional ring (1') has a trimming (5) that rests on the eyeball, the ring (1) that is presently proposed rests on the eyeball by means of an edge immediately adjacent to the aforementioned posterior edge. As we have explained before, and as it is obvious, this improves its adaptation and its capacity to attach itself to the eyeball.

Furthermore, it has also been decided that the body (1) shall include on its front edge, which corresponds to the incarcerated outlet (2), a perimeter stepped expansion (6), that increases the exterior perimeter of the frontal base of the body (1) and which, consequently, increases the length of the classical dovetail guide (7) on which the cutting blade of the microkeratome moves, as represented in the drawings.

Also with the aim of enhancing the length of the dovetail guide (7), it has been decided that the exterior perimeter of the front base of the body or ring (1), instead of adopting the traditional circular configuration shown in figure 3, shall present exterior bulges (8) corresponding to the tips of the aforementioned guide (7), which determine an irregular configuration for this perimeter, which also widens the said guides (7), as emerges from the schematic exploded representation in figure 4.

Therefore, given that the length of the tracks connecting both the suction ring and the cutting blade is increased, it is possible to achieve the objectives of the invention, i.e. on the one hand, to improve the attachment of the suction ring to the eyeball and on the other, to improve the working conditions of the cutting blade on the ring, and consequently, on the eyeball.

## Claims

1. Suction ring for microkeratomes, composed of a ring-shaped body, with a noticeably incarcerated front opening (2) that is attachable to the eyeball, with an anterolateral cannula (4) for connection by means of a flexible tube to a vacuum pump wherein the side of the body (1) adopts a truncated conical configuration diverging towards the posterior area which is attachable to the eyeball, in order to enhance the contact surface with the latter in the aforementioned posterior area, **characterised by** the fact that the body (1) has a dovetail guide (7) on the front, along which a cutting blade is movable, and a stepped expansion (6) on its front edge or area, meaning an increase in its outer perimeter which in turn means the length of the dovetail guide (7) is increased, and that the body (1), presents on the front area, corresponding to the perimeter stepped expansion (6), exterior bulges (8) corresponding to the tips of the guide (7), which means complementary widening of the effective length of the aforementioned guide.

## Patentansprüche

1. Aus einem ringförmigen Körper bestehender Saugring für Mikrokeratome, mit einer merklich eingeschlossenen Frontöffnung (2), die am Augapfel 'andockbar' ist, mit einer anterolateralen Kanüle (4) für den Anschluss an eine Vakuumpumpe über ein biegsames Rohr, in welcher die Seite des Körpers (1) eine abgestumpfte konische Gestalt annimmt, die auf den hinteren Bereich ausläuft, der am Augapfel 'andockbar' ist, um die Kontaktfläche mit letzterem in dem oben genannten hinteren Bereich zu verbessern, der sich **dadurch kennzeichnet, dass** der Körper (1) vom eine Schwalbenschwanzführung (7) aufweist, entlang der sich eine Schneidklinge bewegen kann und eine abgestufte Ausdehnung (6) an seiner Vorderkante oder seinem Vorderbereich, was eine Erhöhung seines Außenumfangs bedeutet, was dann wieder heißt, dass die Länge der Schwalbenschwanzführung (7) zunimmt und dass der Körper (1) im Vorderbereich, der der abgestuften Ausdehnung (6) des Umfangs entspricht, äußere Erhöhungen (8) aufweist, die den Spitzen der Führung (7) entsprechen, was eine zusätzliche Erweiterung der tatsächlichen Länge der oben genannten Führung bedeutet.

## Revendications

1. Un anneau de succion pour microkératomes, constitué par un corps en forme d'anneau, à ouverture frontale (2) sensiblement étranglée qui est adaptable au globe oculaire, doté d'une canule antérolatérale (4) pour la jonction -moyennant un tuyau flexible- à une pompe à vide où la paroi latérale du corps (1) adopte une configuration tronconique divergeant vers la partie arrière que l'on peut adapter au globe oculaire, pour augmenter la surface de contact avec lui dans cette partie arrière, **caractérisé par le fait que** le corps (1) comporte une glissière à queue d'aronde à l'avant, le long de laquelle une lame tranchante se déplace et un élargissement par étages (6) sur son bord ou sa partie avant, d'où l'augmentation de son périmètre extérieur et de la longueur de la glissière à queue d'aronde (7), et que le corps (1) présente à l'avant, autrement dit dans le périmètre élargi par étages (6), des boursouflures extérieures (8) correspondant aux extrémités de la glissière (7), ce qui augmente un peu plus la longueur réelle de cette glissière.
